(19) **Europäisches Patentamt European Patent Office Office européen des brevets**

(11) **EP 3 998 989 B1**

(12) **FASCICULE DE BREVET EUROPEEN**

(45) Date de publication et mention de la délivrance du brevet:
**07.05.2025 Bulletin 2025/19**

(21) Numéro de dépôt: **20742207.2**

(22) Date de dépôt: **13.07.2020**

(51) Classification Internationale des Brevets (IPC):
**A61F 2/16** *(2006.01)*

(52) Classification Coopérative des Brevets (CPC):
**A61F 2/1618; A61F 2/1654;** G02C 2202/20

(86) Numéro de dépôt international:
**PCT/EP2020/069763**

(87) Numéro de publication internationale:
**WO 2021/009125 (21.01.2021 Gazette 2021/03)**

(54) **IMPLANT OCULAIRE DIFFRACTIF À VISION PROCHE ÉLARGIE**

DIFFRAKTIVES AUGENIMPLANTAT MIT VERGRÖSSERTER NAHSICHT

DIFFRACTIVE OCULAR IMPLANT WITH ENLARGED NEAR VISION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.07.2019 FR 1908077**
**02.07.2020 FR 2007000**

(43) Date de publication de la demande:
**25.05.2022 Bulletin 2022/21**

(73) Titulaire: **Cristalens Industrie**
**22300 Lannion (FR)**

(72) Inventeurs:
• **CASTIGNOLES, Fannie**
**22300 Tredrez Locquemeau (FR)**
• **GOBIN, Laure**
**92410 Ville d'Avray (FR)**
• **DELAGE, Denis**
**35440 Dinge (FR)**

(74) Mandataire: **Regimbeau**
**Parc d'affaires Cap Nord A**
**2, allée Marie Berhaut**
**CS 71104**
**35011 Rennes Cedex (FR)**

(56) Documents cités:
WO-A1-03/107076 FR-A1- 3 072 020
US-A1- 2007 182 921

**Description**

**DOMAINE DE L'INVENTION**

La présente invention se situe dans le domaine des implants oculaires.

Elle a plus particulièrement pour objet un implant oculaire diffractif à vision proche élargie.

**ARRIERE PLAN TECHNOLOGIQUE DE L'INVENTION**

La cataracte est la perte de transparence du cristallin naturel. Le cristallin naturel contribue à un tiers de la convergence de la lumière sur l'œil. La chirurgie de la cataracte consiste à enlever le cristallin naturel pour restaurer une vision claire. Dans les années 1950, M. Ridley a implanté les premières lentilles intraoculaires dans le sulcus pour corriger l'aphaquie et restaurer ainsi le tiers de puissance optique manquante. Les lentilles intraoculaires sont désormais implantées dans le sac capsulaire.

Sans accommodation (au repos), les objets situés à distance de loin sont focalisés (nets) sur la rétine d'un œil emmétrope (ni myope, ni hypermétrope, ni astigmate).

Pour visualiser nettement des objets proches (de la distance intermédiaire à la distance de près) le cristallin naturel jeune a la capacité de se déformer Ainsi, sous l'action des muscles ciliaires qui relâchent les fibres zonulaires en se contractant, les rayons de courbure antérieur et postérieur du cristallin naturel augmentent sa puissance optique de plusieurs dioptries.

La perte de cette accommodation vient avec l'âge et apparaît dans tous les cas après une chirurgie de la cataracte au cours de laquelle le cristallin naturel est remplacé par un cristallin artificiel ne possédant pas la capacité de modifier sa puissance en fonction de la distance de vision souhaitée.

Certains implants dits accommodatifs (polymères déformables, optiques mobiles) tentent de redonner une accommodation, mais les résultats ne sont pas satisfaisants aujourd'hui (en partie à cause de la fibrose du sac capsulaire qui, avec le temps, bloque les possibilités de déformation).

Pour pallier cette perte d'accommodation, de nombreux implants multifocaux se sont développés. Ils sont basés sur la division de l'énergie lumineuse sur plusieurs zones de vision. Au début des années 2000, les optiques diffractives ont prouvé leur supériorité en vision de loin et de près par rapport aux optiques réfractives.

Les optiques diffractives permettent de nombreux choix de répartition d'énergie de diffraction en fonction des distances de vision choisies. La qualité optique résultant du profil choisi est définie par sa FTM (fonction de transfert de modulation).

La FTM est un nombre compris entre 0 et 1 (ou 0% et 100%) qui représente le contraste de l'image (l'image peut être non contrastée car elle est défocalisée ou parce que l'énergie est partagée en plusieurs foyers). La FTM « through focus », en abrégé FTM-TF, représente la FTM en fonction de la défocalisation (laquelle est représentée soit en fonction de la distance de vision, soit en fonction de l'addition (1D=1/1m)). La FTM-TF permet de déterminer la profondeur de champ.

Il a été démontré dans la littérature que quel que soit le profil ou la technologie optique, les résultats visuels sont reliés au profil de FTM de "l'IOL" (acronyme de "intraocular lens" pour "lentille intraoculaire" ou "implant") implanté. Ainsi, en moyenne, l'acuité visuelle binoculaire est meilleure que :

- 0 LogMAR (10/10) quand la FTM est de 0.40 ;
- entre 0 et 0.05 LogMAR (9/10) pour une FTM de 0.20 ;
- 0.1 LogMAR (8/10) pour une FTM de 0.15,

quand la FTM est à 3 mm et à 50 cycles/mm, MAR signifiant "Minimum Angle Resolution" pour "angle de résolution minimum".

Les premières optiques diffractives étaient des optiques bifocales avec un pic de FTM pour la vision de loin (Addition 0D) et un autre pic pour la vision de près (addition +3D ou +4D). Ces implants permettent une vision nette pour la lecture (aux alentours de 40 cm) mais les patients doivent porter une correction pour la vision intermédiaire (entre 40 et 90 cm).

Afin de se rapprocher d'une vision idéale (sans correction de lunettes), avec une vision nette et continue à toutes les distances de vision proche (de 40 à 90 cm), plusieurs solutions se sont développées :

- le "Mix & Match" ;
- les "EDOF" ;
- les implants trifocaux.

Le "Mix & Match" :

Le "Mix and Match consiste à utiliser deux implants bifocaux avec des additions différentes pour chaque œil (un œil pour la vision de près, l'autre pour une vision intermédiaire).

L'objectif est de fournir une image contrastée en vision intermédiaire, ce qui n'est pas le cas des implants trifocaux. Les combinaisons +1.75D /+4D et +1.5D/+3D (additions en plan IOL) ont donné des courbes de "defocus" (c'est à dire d'acuité visuelle en fonction de la distance de vision) intéressantes. Mais la limite de cette solution est le confort en vision binoculaire du patient. Afin de préserver un équilibre binoculaire, une différence d'addition de 0.5D a été proposée et la combinaison +2.5D/+3D (additions en plan IOL) a montré des résultats similaires aux trifocaux, excepté dans la zone 1D et 1.5D (en plan cornéen).

Les EDOF (acronyme de "extended depth of focus" or "extended depth of field", c'est-à-dire "profondeur de champ étendue") :

Les implants à profondeur de champ étendue sont des implants bifocaux (ou parfois trifocaux) avec des additions plus faibles afin de permettre une bonne vision intermédiaire. Il faut noter que plus l'addition d'un implant est faible plus grande va être sa profondeur de champ en distance car l'addition est une fonction en "1/x" de la distance. Ces implants permettent un confort pour la vision de l'ordinateur (vision intermédiaire), mais il est nécessaire de porter des verres correcteurs pour la vision de près.

Les implants trifocaux :

Les implants trifocaux sont des "IOLs" diffractifs dont l'énergie est répartie en 3 pics de vision, à savoir de loin, intermédiaire et de près. Ils permettent donc d'apporter une vision nette et une vision intermédiaire là où les implants bifocaux n'avaient qu'une vision de près nette. Actuellement, les implants trifocaux constituent la solution "haut de gamme" la plus aboutie pour aller vers une vision nette de l'infini à de près.

Mais les implants trifocaux présentent trois pics de vision distincts avec une discontinuité du contraste, de la FTM-TF, entre la vision de près et la vision intermédiaire. Sur certains implants trifocaux cette discontinuité est visible sur la FTM-TF à 50 cycles/mm, laquelle représente des tailles intermédiaires de lettres. La vision est donc nette à 40 et 80 cm, mais pas à 60 cm.

Sur d'autres implants, on retrouve cette discontinuité sur la FTM-TF à 100cycles/mm, c'est-à-dire pour des tailles de lettres plus petites.

L'art antérieur d'intérêt vis-à-vis de la présente invention est constitué par les documents FR 3 072 020, US 2007/182921 et WO 03/107076.

L'objectif de la présente invention est de pallier les inconvénients indiqués plus haut et de proposer un implant intraoculaire qui permet d'obtenir une vision nette et continue sur toute la zone de vision proche utile et nécessitant le déchiffrement de caractères de petite taille (lecture d'un ouvrage disposé à 40 cm du lecteur, téléphone portable, tablette, ordinateur portable disposé à 60 cm de l'utilisateur, ordinateur fixe disposé à 80 cm, etc.).

## RESUME DE L'INVENTION

Ainsi, la présente invention est relative à un implant oculaire diffractif selon la revendication 1.

Grâce à l'invention, on gagne en profondeur de champ en vision proche, tout en conservant une bonne vision de loin, sans discontinuité jusqu'aux fréquences spatiales à 100 cycles/mm.

D'autres caractéristiques avantageuses optionnelles de l'invention sont l'objet des revendications dépendantes.

Dans l'ensemble de la présente demande, on entend par l'expression « vision de loin correcte », une vision telle que la FTM soit supérieure à 20% à 50cycles/mm sur une pupille de 3 mm.

Quant à l'expression « vision proche élargie », sa définition est donnée par la formulation « caractérisé par le fait qu'il présente une courbe de transfert de phase en fonction de la distance de vision (en abrégé PTF-TF) avec une absence de discontinuité sur une profondeur de champ d'au moins 1.3D en plan cornéen, c'est-à-dire sur une plage d'additions en plan cornéen d'au moins 1.3D, l'addition en plan cornéen étant entendue comme l'inverse de la distance entre l'objet vu et la cornée, avantageusement supérieure à 1.45D ».

## BREVE DESCRIPTION DES DESSINS

D'autres caractéristiques et avantages de l'invention apparaîtront à la lecture de la description suivante de modes de réalisation de l'invention. Cette description est faite en référence aux dessins annexés dans lesquels :

- la figure 1 est une courbe de FTM-TF d'un implant trifocal du commerce ;
- la figure 2 est une courbe de FTM-TF d'implant bifocal du commerce ;
- la figure 3 est une courbe de FTM-TF d'un implant selon l'invention
- la figure 4 est une vue d'un "demi-profil" d'un implant selon l'invention, étant entendu que sur cette figure, la variabilité des échelettes représentées est due à une apodisation ;
- la figure 5 est une vue d'un "demi-profil" d'un implant selon l'invention, étant entendu que sur cette figure, la variabilité des échelettes représentées est due à une apodisation ;
- la figure 6 est une courbe de FTM-TF d'un implant selon l'invention ;
- la figure 7 est une courbe de FTM-TF d'un implant selon l'invention ;
- la figure 8 est une courbe de FTM-TF d'un implant selon l'invention.

## DESCRIPTION DETAILLEE DE L'INVENTION

Dans ce qui suit et sauf mention contraire, l'addition est indiquée plan cornéen.

Notion de fonction de transfert de phase (PTF) :

Tout système optique peut être représenté par sa "fonction d'étalement du point" (en abrégé "PSF", pour "point spread function").

La PSF représente la répartition spatiale de l'intensité lumineuse dans le plan image d'un système optique, formée à partir d'un point source objet. Plus la PSF est ponctuelle, meilleure est la qualité optique. Cette PSF qui est dans le domaine spatial est très importante car convertie dans le domaine fréquentiel, il s'agit alors de la "fonction de transfert optique" (en abrégé "OTF") qui permet de simuler l'image de n'importe quel objet vu à travers l'optique du système étudié.

Elle s'exprime ainsi :

$$OTF\big(\omega_x, \omega_y\big) = \iint_{-\infty}^{+\infty} PSF(x, y)\, e^{i(x\omega_x + y\omega_y)} dx\, dy$$
$$= Re\left(OTF\big(\omega_x, \omega_y\big)\right) + i\, Im\left(OTF\big(\omega_x, \omega_y\big)\right)$$

Où $\omega_x$, $\omega_y$ représentent les fréquences spatiales dans l'espace de Fourier, x,y les dimensions spatiales dans l'espace réel et Re et Im les parties réelle et imaginaire d'un nombre complexe, i étant la racine carré de -1 dans l'espace des nombres complexes.

La fonction de transfert de modulation est le module de la fonction de transfert optique, c'est-à-dire la racine carrée de ses parties réelle et imaginaire à la puissance 2.

Elle s'exprime ainsi :

$$MTF\big(\omega_x, \omega_y\big) = \sqrt{Re\left(OTF\big(\omega_x, \omega_y\big)\right)^2 + Im\left(OTF\big(\omega_x, \omega_y\big)\right)^2}$$

La fonction de transfert de phase PTF est l'argument de la fonction de transfert optique. Elle s'exprime ainsi :

$$OTF\big(\omega_x, \omega_y\big) = MTF\big(\omega_x, \omega_y\big)\, e^{-i\, PTF(\omega_x, \omega_y)}$$

Pour avoir un confort de vision sans discontinuité sur une large profondeur de champ, il est intéressant de raisonner en termes de "phase" et d'"inversion de phase". En effet, lorsque la FTM-TF devient nulle après un pic puis présente un nouveau pic, cela correspond souvent à une inversion de phase, ce qui signifie que pour une fréquence spatiale donnée (100/cycles/mm dans le cas d'une courbe de FTM-TF à 100/cycles/mm), le signal perceptible est inversé. Ainsi, par exemple, une mire noire sur fond blanc devient blanche sur fond noir.

Dans le cas des IOLs, on peut avoir une inversion de phase pour des fréquences élevées mais aussi en fonction de la défocalisation. Ainsi, pour les implants trifocaux du commerce, entre la vision intermédiaire et la vision de près, l'implant n'est plus assez focalisé et il y a une inversion de phase.

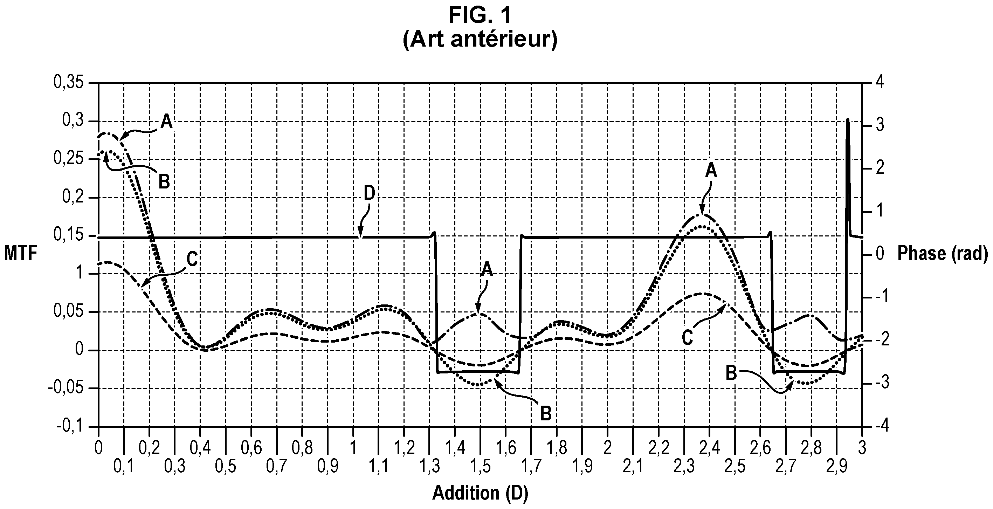

C'est le cas de la figure 1 dans laquelle on a affaire à un implant trifocal du commerce.

Sur cette figure, on trouve, en abscisse, l'addition (de O à 3D en plan cornéen) et en ordonnées la FTM (à gauche) et la phase (à droite).

On y a tracé les courbes suivantes :

- courbe A : courbe de FTM ;
- courbe B : partie réelle de la courbe OTF ;
- courbe C : partie imaginaire de la courbe OTF;
- courbe D : courbe de transfert de phase.

Sauf mention expresse les figures ci-après reprennent les mêmes paramètres.

On observe effectivement une inversion de phase autour de l'addition 1,5D (en plan cornéen). Cet implant ne permet donc pas une vision nette et continue sans inversion de phase entre la vision intermédiaire et la vision de près.

Définition des paramètres auxquels il est fait référence dans la présente demande :

Les courbes de FTM-TF (ou TFFTM = "modulation transfer function through focus") représentent la qualité de l'optique (en % - ou ratio compris entre 0 et 1- ) de contraste de l'image pour un objet de départ ayant un contraste de 100%) en fonction de la distance de vision (décrite en addition en Dioptries : 0 D=vision de loin /+1 à +2D = addition intermédiaire/3 D=addition de près en plan cornéen).

Ce sont des courbes simulées avec le logiciel de simulation optique tel que celui connu sous la marque Zemax pour des implants intraoculaires placés dans un modèle d'œil moyen.

Une courbe de FTM-TF est établie pour une fréquence spatiale donnée. Usuellement, pour les implants multifocaux, on s'intéresse à la FTM-TF à 50 cycles/mm. Des FTM-TF à 25 cycles/mm (objets plus grossiers) et 100 cycles/mm (objets plus fins) sont également intéressantes.

Une courbe de FTM-TF à 50 cycles/mm (par exemple) d'un profil optique donné dépend de la pupille du système optique et de la longueur d'onde de la lumière utilisée.

Ainsi, on s'intéresse préférentiellement à une longueur d'onde verte (546nm) mais il peut également être intéressant de tracer la FTM-TF photopique correspondant à l'intégrale des longueurs d'onde de la lumière du jour, ainsi qu'à la FTM-TF scotopique (vision de nuit).

De même, on s'intéresse préférentiellement à une pupille de 3 mm de diamètre (correspondant à une vision bien éclairée (lecture et vision de jour), mais la continuité de FTM-TF peut être intéressante pour des pupilles de 2 à 6 mm.

Une valeur de FTM supérieure à 0.15 est considérée comme apportant une vision proche satisfaisante au porteur.

La vision de près, VP, est typiquement égale à +3D (en addition plan cornée), mais peut être comprise entre +2D et +4D.

La vision intermédiaire, VI, est typiquement égale à +1.5D (en addition plan cornée), mais peut être comprise entre +1D et +2D.

Implants selon l'invention :

Les implants à vision proche élargie à phase constante selon l'invention peuvent être définis comme suit :
Ce sont des implants diffractifs possédant une vision de loin et une vision proche dont la phase de la fonction de transfert optique est constante sur une zone de vision intermédiaire à proche élargie, c'est à dire sans inversion de phase jusqu'à une fréquence spatiale de 100 cycles/mm, cette fonction de transfert optique étant constante sur une gamme de valeur d'addition qui est au moins 30% (et de préférence 45%) plus large que celle d'un implant bifocal classique, tel que celui dénommé ARtis PL M, commercialisé par le présent déposant.

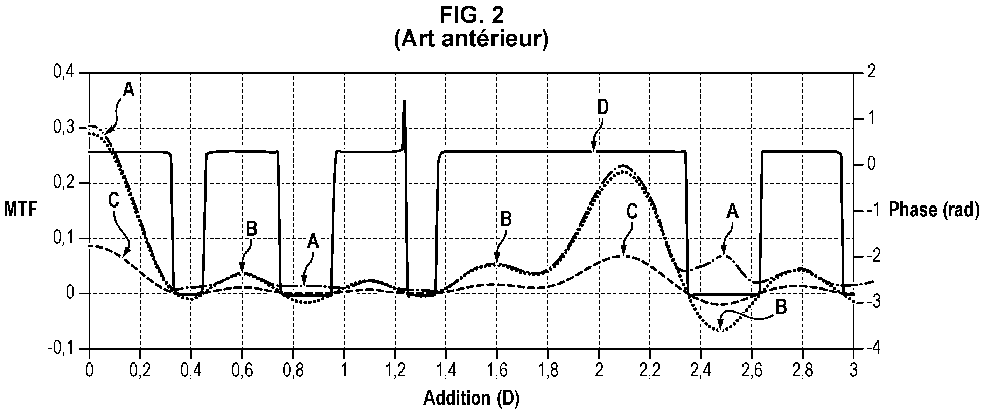

A la figure 2 annexée est représentée la fonction de transfert de phase à 100 cycles/mm en fonction de l'addition pour un implant bifocal « classique » d'addition +3 D.

On constate que la phase est constante (courbe D) sur la zone 1.35 D à 2.3 5D, soit sur une profondeur de 1 D en plan cornéen.

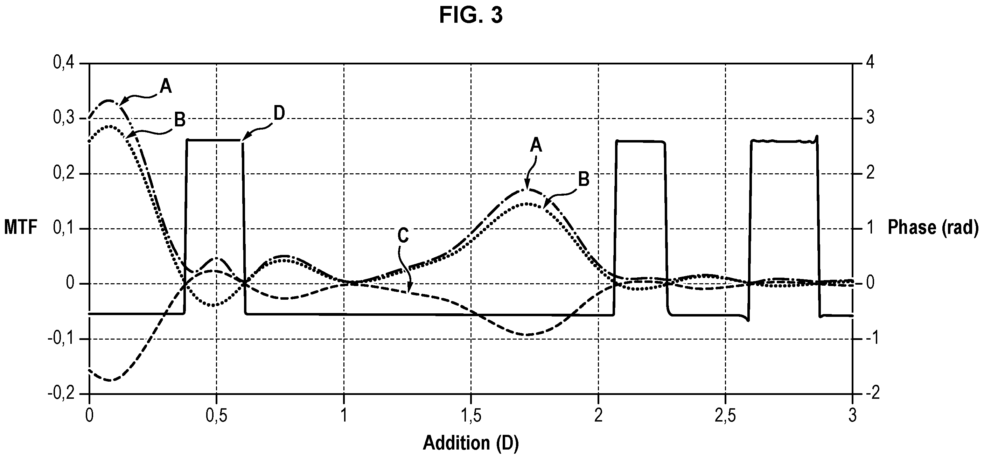

Un exemple de profil à vision proche élargie à phase constante d'un implant conforme à l'invention est représenté à la figure 3.

On constate que la phase est constante sur la zone 0.6D à 2.05 D, soit sur une profondeur de 1.45D en plan cornéen.

De telles courbes peuvent être obtenues à partir de différents implants optiques diffractifs, qui seront décrits ci-après.

On considère bien évidemment qu'un implant de ce type comporte un corps avec au moins une surface optique présentant un axe optique et une pluralité de zones diffractives disposées concentriquement autour de cet axe optique, ces zones concentriques présentant chacune au moins un rayon r.

Pour un implant bifocal, les rayons des anneaux successifs ont pour rayon $r_N$ tel que :

$$r_N = \sqrt{2\,N\,\lambda . f_p}$$

relation dans laquelle :

- N est le numéro de l'anneau (compté à partir du centre) ;
- $f_p$ est la focale correspondant à l'addition pour la vision de près
- $\lambda$ est la longueur d'onde de design (typiquement 546 nm).

La hauteur h des marches diffractives (échelettes) des zones vaut :

$$h = \alpha \frac{\lambda}{\Delta n}$$

relation dans laquelle :

- $\Delta n$ est la variation d'indice de réfraction (c'est à dire la différence entre l'indice de réfraction du matériau de l'implant et celui de l'humeur aqueuse de l'œil, ou encore du milieu environnant quand il s'agit d'un implant intracornéen) ;
- $\alpha$ est le facteur de hauteur du profil.

Si $\alpha$=0.5, alors la répartition d'énergie relative est de 50% pour la vision de loin et 50% pour la vision de près.
La forme des échelettes diffractives de chaque zone est dite "kinoforme" et est décrite par une parabole sur chaque échelette telle que :

$$Profil(x) = \propto . \frac{\lambda}{\Delta n} . \frac{r_N^2 - x^2}{r_N^2 - r_{N-1}^2}$$

Où x est la position radiale.
Selon l'invention, un implant présentant un profil de phase continue entre la vision de près et la vision intermédiaire est réalisé en faisant usage de profils diffractifs donnant une profondeur de champ étendue.
La zone centrale d'un tel implant est définie par un profil dont le rayon des anneaux successifs $r_N$ est fixé par une équation du type :

$$r_N = \sqrt{2\,N\,\lambda\,f_p + 2.\lambda . F2(N) . \Delta_f}$$

expression dans laquelle F2(N) est un polynôme de la variable N d'ordre 3 minimum. F2(N) peut avoir comme expression

$$F2(N) = cte + a.N + b.N^2 + c.N^3 + d.N^4 + \cdots$$

où :

- N est un entier supérieur à 1 ;
- $\lambda$ est la longueur d'onde de conception ;
- $f_p$ est la distance focale correspondant à l'addition pour la vision de près ;
- $\Delta_f$ est la variation de focale ;
- cte est une constante consistant en un nombre réel compris entre -5 et +5 ;
- a, b, c et d sont des nombres réels compris entre -5 et +5.

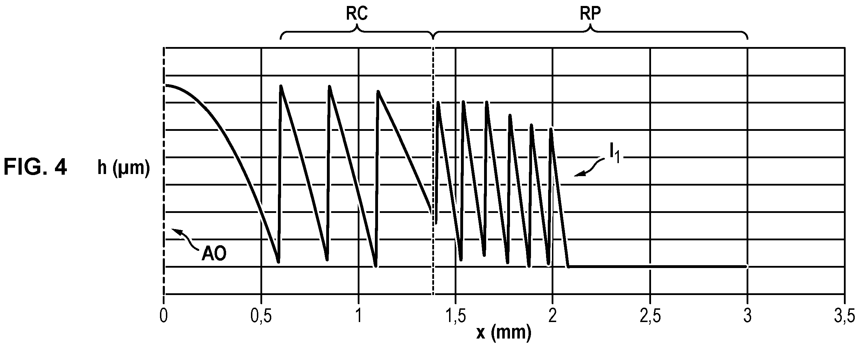

On notera que F2(N) peut être une fonction dont le développement limité ou de Taylor est équivalent du polynôme exprimé ci-dessus. Par le terme « équivalent », on entend que le développement limité ou de Taylor de ladite fonction donne le même résultat que la fonction F2(N) exprimée plus haut.
A titre purement indicatif, ces implants peuvent comporter une région centrale (par exemple de diamètre 1.5 à 6 mm) à profondeur de champ étendue et éventuellement une région zone « périphérique » que l'on peut qualifier de classique (par exemple de 2 à 6 mm de diamètre).
A la figure 4 est représenté le "demi" profil d'un implant I1 conforme à la présente invention.

Par "demi" profil, on entend le fait que l'axe des ordonnées, qui traduit la hauteur des échelettes de ces implants en micromètres, est confondu avec leur axe optique AO et que l'on a représenté uniquement le profil qui s'étend d'un côté de cet axe.

Sur cette figure, lesdites régions centrale et périphérique sont respectivement référencées RC et RP.

On notera ici que le profil de la quatrième échelette visible sur cette figure a été coupé, en raison du commencement de la région périphérique RP.

Bien que « kinoformes », les échelettes pourraient avoir une forme différente (sinus, cosinus par exemple), cette forme différente ne modifiant pas drastiquement la présente invention.

Dans le cas présenté ici, les différentes zones diffractives ont un contour circulaire. Toutefois, selon des modes alternatifs de réalisation, non représentés ici, ces zones diffractives ont un contour en forme d'ellipse dont $r_N$ est le petit rayon.

Toujours selon le mode de réalisation présenté ici, la région centrale RC est à profondeur de champ étendu, présente un rayon d'au moins un millimètre et est entourée par une région périphérique RP réfractive ou diffractive, monofocale ou multifocale, par exemple d'équation bifocale :

$$r_N = \sqrt{2\,N\,\lambda\,.\,f_p}\,.$$

A contrario, ce pourrait être l'inverse, de sorte que ce serait la région centrale RC, présentant un rayon d'au moins un millimètre, qui serait réfractive ou diffractive, monofocale ou multifocale, par exemple d'équation bifocale :

$$r_N = \sqrt{2\,N\,\lambda\,.\,f_p}$$

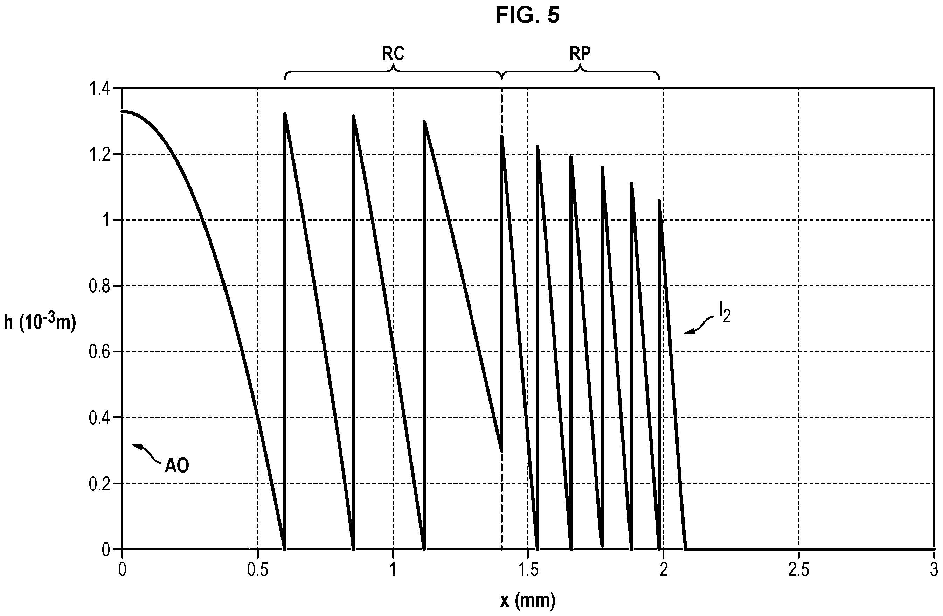

A la figure 5 est représenté le "demi-profil" d'un autre implant I2 conforme à la présente invention.

Les paramètres principaux d'implants selon l'invention sont donnés ci-après.

<u>Exemple 1</u> :

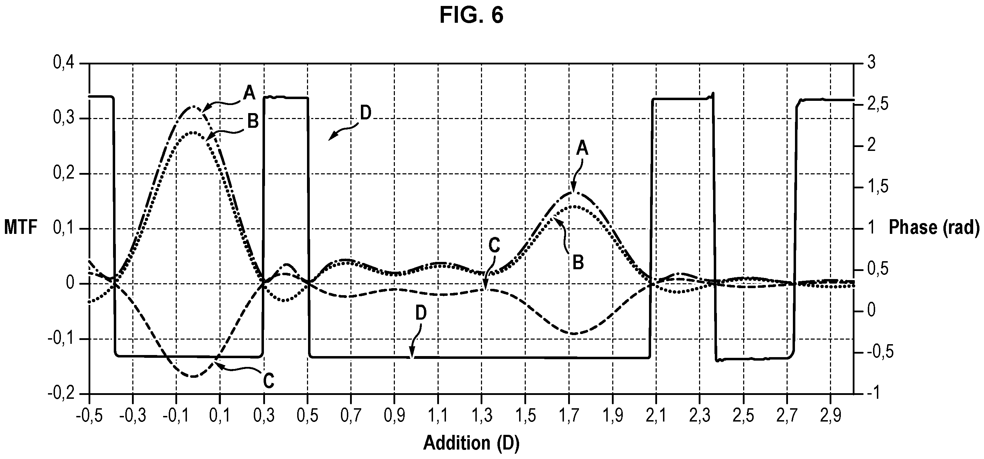

Fonction F(N) d'ordre 4 (qui correspond au profil de la figure 5 et aux courbes de la figure 6) :

Tableau 1

| Région centrale : anneaux 1 à 4 | $f_{p1}$ | 1000/3.05 |
|---|---|---|
| | $\propto_1$ | 0.5 |
| | $\Delta_f$ | 800 |
| Région périphérique : anneaux 6 à 12 | $f_{p2}$ | 1000/2.77 |
| | $\propto_2$ | 0.5 |

• Région centrale :

$$r1_N = \sqrt{2\,\lambda\,.\,\text{N}\,.\,f_{p1} + 2\,\lambda\,.\left(\text{N}.(\tfrac{N-1}{5})^3\right).\Delta_f}$$

$$Profil_1(x) = \propto_1 . \frac{\lambda}{\Delta n} . \frac{r1_N^2 - x^2}{r1_N^2 - r1_{N-1}^2}$$

• Région périphérique :

$$r2_N = \sqrt{2\,(N)\,\lambda\,.\left(f_{p2}\right)}$$

$$Profil_2(x) = \propto_2 . \frac{\lambda}{\Delta n} . \frac{r2_N^2 - x^2}{r2_N^2 - r2_{N-1}^2}$$

Ainsi qu'on le constate sur la figure 6, la phase est constante sur la zone de 0.5D à 2.05 soit sur une profondeur de 1.55D en plan cornéen.

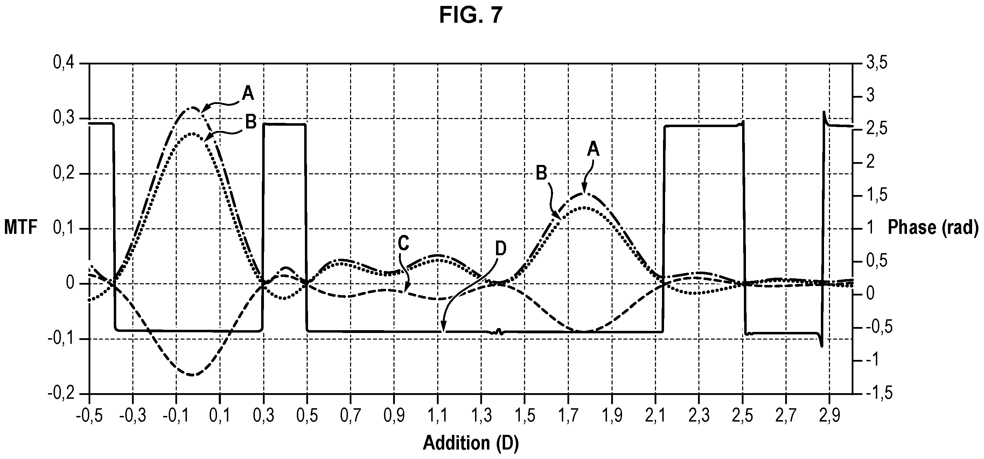

**Exemple 2** (qui correspond au profil de la figure 4 et aux courbes de la figure 7)

Fonction F(N) d'ordre 5 :

Tableau 2

| Région centrale : anneaux 1 à 4 | $f_{p1}$ | 1000/3.05 |
|---|---|---|
| | $\propto_1$ | 0.5 |
| | $\Delta_f$ | 1400 |
| Région périphérique anneaux 6 à 12 | $f_{p2}$ | 1000/2.77 |
| | $\propto_2$ | 0.5 |

- région centrale :

$$r1_N = \sqrt{2\,\lambda.\,\text{N}.f_{p1} + 2\,\lambda.\left(\text{N}.(\tfrac{N-1}{5})^4\right).\Delta_f}$$

$$Profil_1(x) = \propto_1.\frac{\lambda}{\Delta n}.\frac{r1_N^2 - x^2}{r1_N^2 - r1_{N-1}^2}$$

- région périphérique :

$$r2_N = \sqrt{2\,(N)\,\lambda.\left(f_{p2}\right)}$$

$$Profil_2(x) = \propto_2.\frac{\lambda}{\Delta n}.\frac{r2_N^2 - x^2}{r2_N^2 - r2_{N-1}^2}$$

Ainsi qu'on le constate sur la figure 7, la phase est constante sur la zone de 0.5D à 2.14D soit sur une profondeur de 1.64D au plan cornéen.

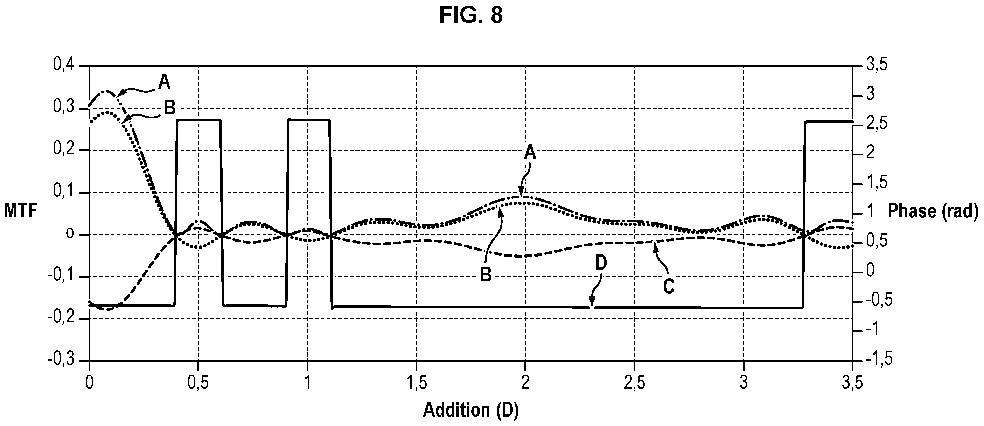

Enfin, dans le mode de réalisation de la figure 8, la phase est constante sur la zone de 1.1D à 3.3D, soit sur une profondeur de 2.2 en plan cornéen.

Comme indiqué plus haut, les profils diffractifs (encore appelés marches ou échelettes) des implants selon l'invention peuvent être apodisés (c'est-à-dire qu'on a affaire à réduction de la hauteur des marches entre le centre et la périphérie) en fonction du rayon (qui constitue la variable x), suivant l'équation suivante :

**Profil Apodisé (x) = Profil (x) * Apodisation(x)**

La fonction « apodisation(x) » est une fonction décroissante telle que, pour 0<abs(x)<r max (r max = rayon maximal du profil diffractif), alors 0<Apodisation(x) ≤1.

Par exemple, cette fonction peut prendre la forme suivante : Apodisation (x)= (1- abs(a.x/b)^c) avec a, b et c consistant en des nombres réels.

Le profil diffractif peut être composé de marches (échelettes) diffractives concentriques, circulaires ou ovales. Dans d'autres modes de réalisation l'effet diffractif peut être obtenu par l'alternance de zones pleines et de zones vides (trous, fentes), ce qui modifie l'indice de réfraction local par zones et génère de la diffraction de la même manière que les échelettes.

Exprimé différemment, ce profil diffractif peut être défini non pas par une forme géométrique, mais par une variation d'indices de réfraction du ou des matériaux qui le composent et qui vont créer le même effet. La modification d'indice de réfraction peut par exemple être obtenue par l'alternance de zones pleines d'indice « n mat » et de zones vides d'indice « n0 » (constituées de trous, de fentes), ce qui modifie l'indice de réfraction local par zones et génère de la diffraction de la même manière que les échelettes.

Les implants selon l'invention permettent de corriger la presbytie. Ils peuvent permettre également de corriger les autres amétropies (myopie, hypermétropie, astigmatisme).

Il peut s'agir d'implants intracornéens (lenticules), de chambre antérieure (phaque ou pseudophaque), ou de chambre postérieure intraoculaire ou de sulcus).

## Revendications

**1.** Implant oculaire diffractif à vision de loin correcte, c'est-à-dire une vision telle que la fonction de transfert de modulation (FTM) dudit implant soit supérieure à 20% à 50 cycles/mm sur une pupille de 3mm, et à vision proche élargie (I1, I2), c'est-à-dire avec une absence de discontinuité de sa courbe de transfert de phase en fonction de la distance de vision (en abrégé PTF-TF), **caractérisé par le fait que** :

- il présente une courbe de transfert de phase en fonction de la distance de vision (PTF-TF) avec une absence de discontinuité sur une profondeur de champ d'au moins 1.3D en plan cornéen, c'est-à-dire sur une plage d'additions en plan cornéen d'au moins 1.3D, l'addition en plan cornéen étant entendue comme l'inverse de la distance entre l'objet vu et la cornée, avantageusement supérieure à 1.45D, laquelle absence de discontinuité se situe entre la vision intermédiaire et la vision proche, c'est à dire entre 0.5D et 4D pour des fréquences spatiales de 0 à 100 cycles/mm, ceci pour une pupille d'au moins 3 mm de diamètre,
- il comporte un corps avec au moins une surface optique présentant un axe optique (AO) et une pluralité de zones diffractives disposées concentriquement autour dudit axe optique (AO), ces zones concentriques présentant chacune au moins un rayon r et étant réparties entre une région centrale (RC) et une région périphérique (RP),au moins une région centrale (RC) ou périphérique (RP) desdites zones diffractives présentant un profil de N échelettes successives dont les rayons $r_N$ successifs, au fur et à mesure que l'on s'éloigne dudit axe optique (AO), répondent à la relation :

$$r_N = \sqrt{2\,N\,\lambda\,f_p + 2.\lambda.F2(N).\Delta_f}$$

relation dans laquelle :

N est un entier supérieur ou égal à 1 et prenant les valeurs entières successives jusqu'à N max, N max valant au minimum 4 ;
$\lambda$ est la longueur d'onde de conception ;
$f_p$ est la distance focale correspondant à l'addition pour la vision de près ;
$\Delta_f$ est la variation de focale, qui est non nulle, positive ou négative, et dont la valeur absolue est inférieure à 10000 ; ;
F2(N) est un polynôme de la variable N d'ordre compris entre 3 et 5, qui s'exprime comme suit :

$$F2(N) = cte + a.N + b.N^2 + c.N^3 + d.N^4 + \cdots \qquad ,$$

- et que la hauteur maximale desdites échelettes successives, c'est à dire des marches diffractives, à savoir la différence entre deux échelettes successives, est donnée par la relation :

$$h = \alpha\,\frac{\lambda}{\Delta n}$$

relation dans laquelle :

- $\Delta n$ est la variation d'indice de réfraction, c'est à dire la différence entre l'indice de réfraction du matériau de l'implant et celui de l'humeur aqueuse de l'œil ou du milieu environnant ;
- $\alpha$ est le facteur de hauteur de l'échelette, compris entre 0.25 et 1.75.

**2.** Implant (I1, I2) selon la revendication 1, **caractérisé par le fait que** "cte" est un nombre réel compris entre -5 et +5.

3. Implant (I1, I2) selon la revendication 1 ou 2, **caractérisé par le fait que** a, b, c, d, etc. sont des nombres réels compris entre -5 et +5.

4. Implant (I1, I2) selon l'une des revendications 1 à 3, **caractérisé par le fait que** lesdites zones diffractives ont un contour circulaire.

5. Implant (I1, I2) selon l'une des revendications 1 à 3, **caractérisé par le fait que** lesdites zones diffractives ont un contour en forme d'ellipse dont $r_N$ est le petit rayon.

6. Implant (I1, I2) selon l'une des revendications 1 à 3, **caractérisé par le fait que** lesdites zones diffractives sont constituées par l'alternance de zones pleines d'indice « nmat » et de zones vides d'indice « n0 », lesdites zones vides consistant en des fentes ou en des trous.

7. Implant (I1, I2) selon l'une des revendications 1 à 6, **caractérisé par le fait que** ladite courbe est sans discontinuité à partir de 0.8D en plan cornéen.

8. Implant (I1, I2) selon l'une des revendications 1 à 6, **caractérisé par le fait que** ladite courbe est sans discontinuité à partir de 2D en plan cornéen.

9. Implant (I1, I2) selon l'une des revendications 1 à 8, **caractérisé par le fait que** ladite région est une région centrale (RC) qui présente un rayon d'au moins un millimètre et qui est entourée par une région périphérique (RP) réfractive ou diffractive, monofocale ou multifocale, par exemple d'équation bifocale : $r_N = \sqrt{2\,N\,\lambda.f_p}$ .

10. Implant (I1, I2) selon l'une des revendications 1 à 8, **caractérisé par le fait que** ladite région est une région périphérique (RP), laquelle ceinture une région centrale (RC), cette dernière présentant un rayon d'au moins un millimètre et étant réfractive ou diffractive, monofocale ou multifocale, par exemple d'équation bifocale : $r_N = \sqrt{2\,N\,\lambda.f_p}$ .

11. Implant (I1, I2) selon l'une des revendications 1 à 10, **caractérisé par le fait que** la fonction F2(N) est un polynôme de la variable N d'ordre 3.

12. Implant (I1, I2) selon l'une des revendications précédentes, **caractérisé par le fait qu'**il est choisi dans le groupe suivant : implant intra cornéen, de chambre antérieure (phaque ou pseudophaque), implant de chambre postérieure intraoculaire ou de sulcus.

13. Implant (I1, I2) selon l'une des revendications précédentes, **caractérisé par le fait qu'**il présente une surface asphérique.

14. Implant (I1, I2) selon l'une des revendications 1 à 5 et 7 à 13, **caractérisé par le fait qu'**il présente un profil apodisé, c'est-à-dire que la hauteur desdites échelettes diminue au fur et à mesure que l'on s'éloigne dudit axe optique, afin de limiter le phénomène de halo en vision nocturne.

**Patentansprüche**

1. Diffraktives Augenimplantat mit korrekter Fernsicht, das heißt einer Sicht, solchen dass die Modulationsübertragungsfunktion (MTF) des genannten Implantats bei 50 Zyklen/mm auf einer 3mm-Pupille größer als 20% ist, und mit erweiterter Nahsicht (I1, I2), d.h. mit einer fehlenden Diskontinuität seiner Phasenübertragungskurve in Abhängigkeit von der Sehentfernung (abgekürzt PTF-TF), **dadurch gekennzeichnet, dass**:

   - es eine Phasenübertragungskurve als Funktion der Betrachtungsentfernung (PTF-TF) mit einer Diskontinuitätsfreiheit über eine Schärfentiefe von mindestens 1.3D in der Hornhautebene, das heißt über einen Bereich von Additionen in der Hornhautebene von mindestens 1.3D, wobei die Addition in der Hornhautebene als der Kehrwert des Abstands zwischen dem gesehenen Objekt und der Hornhaut verstanden wird , vorteilhafterweise größer als 1.45D, wobei diese Diskontinuitätsfreiheit zwischen Zwischen- und Nahsicht, das heißt zwischen 0.5D und 4D bei Ortsfrequenzen von 0 bis 100 Zyklen/mm liegt, dies für eine Pupille mit einem Durchmesser von mindestens 3 mm,

- es einen Körper mit mindestens einer optischen Oberfläche umfasst, die eine optische Achse (AO) und eine Vielzahl von diffraktiven Zonen aufweist, die konzentrisch um die genannte optische Achse (AO) angeordnet sind, wobei diese konzentrischen Zonen jeweils mindestens einen Radius r aufweisen und zwischen einem zentralen Bereich (RC) und einem peripheren Bereich (RP) aufgeteilt sind, wobei mindestens ein zentraler (RC) oder peripherer (RP) Bereich der genannten diffraktiven Zonen ein Profil von N aufeinanderfolgenden kleinen Leitern aufweist, deren aufeinanderfolgende Radien $r_N$, je weiter man sich von der genannten optischen Achse (AO) entfernt, der folgenden Beziehung entsprechen:

$$r_N = \sqrt{2\,N\,\lambda\,f_p + 2.\lambda.F2(N).\Delta_f}$$

Beziehung, in der:

N ist eine ganze Zahl größer oder gleich 1 und nimmt aufeinanderfolgende ganzzahlige Werte bis N max an, wobei N max mindestens 4 beträgt;
$\lambda$ ist die Entwurfswellenlänge ;
$f_p$ ist die Brennweite, die der Addition für die Nahsicht entspricht ;
$\Delta_f$ ist die Brennweitenänderung , die nicht Null, positiv oder negativ ist,
und deren absoluter Wert kleiner als 10000 ist; ;
F2(N) ist ein Polynom der Variablen N mit einer Ordnung zwischen 3 und 5, das wie folgt ausgedrückt wird:

$$F2(N) = cte + a.N + b.N^2 + c.N^3 + d.N^4 + \cdots \ ,$$

- und dass die maximale Höhe der genannten aufeinanderfolgenden kleinen Leitern, das heißt der beugenden Stufen, das heißt die Differenz zwischen zwei aufeinanderfolgenden kleinen Leitern, durch die Beziehung gegeben ist :

$$h = \alpha\,\frac{\lambda}{\Delta n}$$

Beziehung, in der:

- $\Delta n$ ist die Änderung des Brechungsindex, d. h. der Unterschied zwischen dem Brechungsindex des Implantatmaterials und dem des Kammerwassers des Auges oder des umgebenden Mediums ;
- $\alpha$ ist der Höhenfaktor der kleinen Leiter, der zwischen 0,25 und 1,75 liegt.

2. Implantat (I1, I2) nach Anspruch 1, **dadurch gekennzeichnet, dass** "cte" eine reelle Zahl zwischen -5 und +5 ist.

3. Implantat (I1, I2) nach Anspruch 1 oder 2, **dadurch gekennzeichnet, dass** a, b, c, d und so weiter reelle Zahlen zwischen -5 und +5 sind.

4. Implantat (I1, I2) nach einem der Ansprüche 1 bis 3, **dadurch gekennzeichnet, dass** die genannten diffraktiven Zonen eine kreisförmige Kontur aufweisen.

5. Implantat (I1, I2) nach einem der Ansprüche 1 bis 3, **dadurch gekennzeichnet, dass** die genannten diffraktiven Zonen eine Kontur in Form einer Ellipse haben, deren kleiner Radius $r_N$ ist.

6. Implantat (I1, I2) nach einem der Ansprüche 1 bis 3, **dadurch gekennzeichnet, dass** die genannten diffraktiven Zonen durch die Abwechslung von vollen Zonen mit dem Index "nmat" und leeren Zonen mit dem Index "n0" gebildet werden, wobei die genannten leeren Zonen aus Schlitzen oder Löchern bestehen.

7. Implantat (I1, I2) nach einem der Ansprüche 1 bis 6, **dadurch gekennzeichnet, dass** die genannte Kurve ab 0,8D in der Hornhautebene ohne Unterbrechung verläuft.

8. Implantat (I1, I2) nach einem der Ansprüche 1 bis 6, **dadurch gekennzeichnet, dass** die genannte Kurve ohne Unterbrechung von 2D in der Hornhautebene verläuft.

9. Implantat (I1, I2) nach einem der Ansprüche 1 bis 8, **dadurch gekennzeichnet, dass** der genannte Bereich ein zentraler Bereich (RC) ist, der einen Radius von mindestens einem Millimeter aufweist und von einem refraktiven oder diffraktiven, monofokalen oder multifokalen peripheren Bereich (RP) umgeben ist, zum Beispiel mit der bifokalen Gleichung: $r_N = \sqrt{2\,N\,\lambda.f_p}$ .

10. Implantat (I1, I2) nach einem der Ansprüche 1 bis 8, **dadurch gekennzeichnet, dass** der genannte Bereich ein peripherer Bereich (RP) ist, der einen zentralen Bereich (RC) umschließt, wobei letzterer einen Radius von mindestens einem Millimeter aufweist und refraktiv oder diffraktiv, monofokal oder multifokal ist, zum Beispiel mit der bifokalen Gleichung: $r_N = \sqrt{2\,N\,\lambda.f_p}$ .

11. Implantat (I1, I2) nach einem der Ansprüche 1 bis 10, **dadurch gekennzeichnet, dass** die Funktion F2(N) ein Polynom der Variablen N der Ordnung 3 ist.

12. Implantat (I1, I2) nach einem der vorhergehenden Ansprüche, **dadurch gekennzeichnet, dass** es aus der folgenden Gruppe ausgewählt ist: Intrahornhaut-, Vorderkammer- (phakisch oder pseudophakisch), intraokulare Hinterkammer- oder Sulkusimplantat.

13. Implantat (I1, I2) nach einem der vorhergehenden Ansprüche, **dadurch gekennzeichnet, dass** es eine asphärische Oberfläche aufweist.

14. Implantat (I1, I2) nach einem der Ansprüche 1 bis 5 und 7 bis 13, **dadurch gekennzeichnet, dass** es ein apodisiertes Profil aufweist, das heißt, dass die Höhe der genannten kleinen Leitern mit zunehmender Entfernung von der optischen Achse abnimmt, um das Halo-Phänomen bei der Nachtsicht zu begrenzen.

## Claims

1. A diffractive ocular implant with correct distance vision, i.e., a vision such that the modulation transfer function (MTF) of said implant is greater than 20% at 50 cycles/mm on a 3mm pupil, and enlarged near vision (I1, I2), i.e., with an absence of discontinuity of its phase transfer curve as a function of the viewing distance (abbreviated PTF-TF), **characterized by** the fact that:

   - it has a phase transfer curve as a function of the viewing distance (PTF-TF) with an absence of discontinuity over a depth of field of at least 1.3D in the corneal plane, i.e., over an area of additions in the corneal plane of at least 1.3D, the addition in the corneal plane being understood as the inverse of the distance between the object viewed and the cornea, advantageously greater than 1.45D, which absence of discontinuity is located between intermediate vision and near vision, i.e. between 0.5D and 4D for spatial frequencies from 0 to 100 cycles/mm for a pupil of at least 3 mm in diameter,
   - it comprises a body with at least one optical surface having an optical axis (AO) and a plurality of diffractive zones arranged concentrically around said optical axis (AO), these concentric zones each having at least one radius r and being distributed between a central region (RC) and a peripheral region (RP), at least one central region (RC) or peripheral region (RP) of said diffractive zones having a profile of N successive echelettes, the successive $r_N$ of which, as one moves away from said optical axis (AO), respond to the relation:

$$r_N = \sqrt{2\,N\,\lambda\,f_p + 2.\lambda.F2(N).\Delta_f}$$

   relation wherein:

   N is an integer greater than or equal to 1 and taking successive integer values up to N max, wherein N max is at least 4 ;
   $\lambda$ is the conception wavelength;
   $f_p$ is the focal length corresponding to the addition for near vision;

$\Delta_f$ is the focal length variation;, which is not zero, positive or negative, and whose absolute value is less than 10,000;

F2(N) is either a polynomial of the variable N of order between 3 and 5, which is expressed as follows:

$$F2(N) = cte + a.N + b.N^2 + c.N^3 + d.N^4 + \cdots ,$$

- and that the maximum height h of said successive echelettes (diffractive steps) being given by the relation:

$$h = \alpha \frac{\lambda}{\Delta n}$$

relation wherein:

- $\Delta n$ is the refractive index variation, i.e., the difference between the refractive index of the implant material and that of the aqueous humor of the eye or the surrounding environment;
- $\alpha$ is the height factor of the echelette, comprised between 0.25 and 1.75.

2. The implant (I1, I2) according to claim 1, **characterized by** the fact that "cte" is a real number comprised between -5 and +5.

3. The implant (I1, I2) according to claim 1 or 2, **characterized by** the fact that a, b, c, d etc. are real numbers comprised between -5 and +5.

4. The implant (I1, I2) according to one of claims 1 to 3, **characterized by** the fact that said diffractive zones have a circular contour.

5. The implant (I1, I2) according to one of claims 1 to 3, **characterized by** the fact that said diffractive zones have an elliptical contour of which $r_N$ is the small radius.

6. The implant (I1, I2) according to one of claims 1 to 3, **characterized by** the fact that said diffractive zones are made up by alternating full zones of an index "nmat" and empty zones of an index "n0", said empty zones consisting of slits or holes.

7. The implant (I1, I2) according to one of claims 1 to 6, **characterized by** the fact that said curve has no discontinuity from 0.8D in the corneal plane.

8. The implant (I1, I2) according to one of claims 1 to 6, **characterized by** the fact that said curve has no discontinuity from 2D in the corneal plane.

9. The implant (I1, I2) according to one of claims 1 to 8, **characterized by** the fact that said region is a central region (RC) which has a radius of at least one millimeter and which is surrounded by a peripheral region (PR) which is refractive or diffractive, monofocal or multifocal, for example with a bifocal equation: $r_N = \sqrt{2\,N\,\lambda.f_p}$ .

10. The implant (I1, I2) according to one of claims 1 to 8, **characterized by** the fact that said region is a peripheral region (RP), which surrounds a central region (RC), the latter having a radius of at least one millimeter and being refractive or diffractive, monofocal or multifocal, for example with a bifocal equation: $r_N = \sqrt{2\,N\,\lambda.f_p}$ .

11. The implant (I1, I2) according to one of claims 1 to 10, **characterized by** the fact that function F2(N) is a polynomial of the variable N of order 3.

12. The implant (I1, I2) according to one of the preceding claims, **characterized by** the fact that it is chosen in the following group: intracorneal implant, anterior chamber (phakic or pseudophakic), posterior intraocular chamber or sulcus implant.

13. The implant (I1, I2) according to one of the preceding claims, **characterized by** the fact that it has an aspherical surface.

14. The implant (I1, I2) according to one of claims 1 to 5 and 7 to 13, **characterized by** the fact that it has an apodized profile, i.e., the height of said echelettes decreases as one moves away from said optical axis, in order to limit the halo phenomenon in night vision.

FIG. 1
(Art antérieur)

FIG. 2
(Art antérieur)

FIG. 3

EP 3 998 989 B1

FIG. 4

FIG. 5

EP 3 998 989 B1

# FIG. 6

EP 3 998 989 B1

# FIG. 7

EP 3 998 989 B1

FIG. 8

## RÉFÉRENCES CITÉES DANS LA DESCRIPTION

**Documents brevets cités dans la description**

- FR 3072020
- US 2007182921 A
- WO 03107076 A